# EUROPÄISCHE PATENTSCHRIFT

(11) **EP 3 426 173 B1**
(45) Veröffentlichungstag und Bekanntmachung des Hinweises auf die Patenterteilung: **05.08.2020**
(21) Anmeldenummer: 17709112.1
(22) Anmeldetag: 10.03.2017
(51) Int. Cl.: A61B 17/68, A61F 2/28, A61F 2/30

(54) **VERFAHREN ZUM BEREITSTELLEN VON TEILELEMENTEN EINES MEHRTEILIGEN IMPLANTATS ODER EINER MEHRTEILIGEN OSTEOSYNTHESE**
METHOD FOR PROVIDING PARTS OF A MULTI-PART IMPLANT OR MULTI-PART OSTEOSYNTHESIS
PROCEDE DE PREPARATION DE PARTIES D'UN IMPLANT EN PLUSIEURS PARTIES OU D'UNE OSTEOSYNTHESE EN PLUSIEURS PARTIES

(30) Priorität: 11.03.2016 EP 16159953
(43) Veröffentlichungstag der Anmeldung: 16.01.2019
(73) Patentinhaber: Universität Basel, Vizerektorat Forschung, 4003 Basel (CH)
(72) Erfinder: STÜBINGER, Stefan, 4102 Binningen BL (CH); ZILLIG, Timo, 4310 Rheinfelden AG (CH)
(74) Vertreter: Kleine, Hubertus
(86) Internationale Anmeldenummer: PCT/EP2017/055640
(87) Internationale Veröffentlichungsnummer: WO 2017/153560

(56) Entgegenhaltungen:
- US-A1- 2012 330 427
- US-A1- 2013 289 727
- US-A1- 2015 088 142

## Beschreibung

Die vorliegende Erfindung betrifft ein Verfahren zum Bereitstellen von Teilelementen eines mehrteiligen Implantats oder einer mehrteiligen Osteosynthese und ein mehrteiliges Implantat und/oder eine mehrteilige Osteosynthese.

Mehrteilige Implantate sind aus der US 6 060 641 A1, aus der US 2015/0018829 A1 und aus der WO 2008/051967 A2 bekannt. Diese sind aus mehreren Einzelteilen zusammengesetzt und teilweise flexibel und teilweise starr entlang eines Knochengerüstes angeordnet. Nachteilig daran ist, dass diese Implantate nicht speziell auf den Patienten abgestimmt sind. Zwar sind die Implantate durch Entnahme oder Hinzufügen von Teilelementen in ihrer Größe variabel, jedoch geschieht dies stets nur innerhalb der Grenzen welche durch die Dimensionierung der jeweiligen Teilelemente vorgegeben ist. Dadurch sind solche Implantate nicht ideell auf den einzelnen Patienten angepasst, sondern stets je nach Anzahl der Teilelemente ein wenig zu groß oder ein wenig zu klein.

Die US 2012/0330427 A1 offenbart ein mehrteiliges Implantat, beispielsweise zwei Kieferabschnitte, welche über eine Steckverbindung miteinander verbunden sind. Sie weisen Anschlüsse auf zur Positionierung auf bzw. über einer Kinnknochenfraktur auf und dienen der Stabilisierung dieses Knochens. Anhand eines CT-Scans kann eine Dimensionierung artgleicher Verbindungsmittel aufgrund der Dicke eines Knochens vorgenommen werden. Der CT-Scan wird dabei nur zur Oberflächenanalyse des Knochens und zur Fertigung einer zur Stützung des Knochens patientenindividualisierten angepassten Stützschiene verwendet. Die Nutzung eines CT-Scans erstreckt sich somit auf die Fertigung von oberflächenangepassten patientenindividualisierten Stützschienen und der Dimensionierung der Verbindungsmittel zwischen den Stützschienen. Das Material der Stützschienen kann dabei Polyethylenmaterial oder biokompatibles Material sein.

Die US 2013/0289727 A nutzt einen CT-Scan zur Dimensionierung von Implantationsplatten. Die Art der Verbindungsmittel zwischen den Implantationsplatten ist dabei ein überlappender Flansch, welcher mittels einem Befestigungsmittel festgelegt wird. Das Material der Platten ist dabei stets gleich.

Um einen optimalen Sitz eines Implantats und/oder eines Knochengerüstes zu ermöglichen ist es daher Aufgabe der vorliegenden Erfindung ein Verfahren bereitzustellen, mit welchem ein individuell auf den Patienten abgestimmtes Implantat oder ein individuell auf den Patienten abgestimmte Osteosynthese fertigbar ist.

Die Erfindung löst diese Aufgabe durch ein Verfahren mit den Merkmalen des Anspruchs 1.

Ein erfindungsgemäßes Verfahren zum Bereitstellen von Teilelementen eines mehrteiligen Implantats oder einer mehrteiligen Osteosynthese vor deren Einführung in einen menschlichen und/oder tierischen Körper umfasst die folgenden Schritte:
A) Erfassen von Daten eines Patienten, für welchen das Implantat und/oder die Osteosynthese bestimmt ist;
   Als Osteosynthese ist die Gesamtheit aus Verbindungsmitteln einer Verbindung zu verstehen, welche zwischen zwei oder mehr Knochen oder Knochenfragmente eingesetzt werden kann mit dem Ziel, dass diese zusammenwachsen.
   Diese Daten sind erfindungsgemäß Aufnahmen eines CT-Scans. Üblicherweise werden diese Aufnahmen zur Diagnose genutzt. Im vorliegenden Fall werden diese Aufnahmen jedoch zur Erstellung eines Modells unabhängig von einer Diagnose erstellt oder alternativ nach erfolgter Diagnose weiterverwandt.
B) Erstellen eines Modells anhand der erfassten Daten;
   Das Erstellen des Modells kann als Computermodell erfolgen. Hierfür kann vorzugsweise ein Computerprogramm genutzt werden, welches auf der Finite-Elemente-Methode beruht. Entsprechende Computerprogramme sind für andere Anwendungen bereits käuflich erhältlich und müssen lediglich für den Anwendungsfall in an sich bekannter Weise umgestellt werden.
C) Erstellen von Fertigungsvorgaben für zumindest zwei oder mehr zu einem Implantat und/oder zu einer Osteosynthese zusammensetzbaren Teilelementen auf Grundlage des erstellten Modells wobei die Fertigungsvorgaben umfassen: C1) eine Dimensionierung der Teilelemente
   Die Fertigungsvorgaben werden vorzugsweise anhand des Computermodells derart erstellt, dass die Teilelemente je nach ihrer Funktion unterschiedlich dimensioniert werden. Auch eine Knochenplatte ist an einer Stelle etwas dicker als an einer anderen. Die benötigte Materialdicke und Flexibilität der Teilelemente kann durch das Modell, welches auf patientenspezifischen Daten beruht, individuell ausgestaltet und gefertigt werden.
D) Fertigung der Teilelemente auf Basis der Fertigungsvorgaben; wobei die Teilelemente miteinander zu einem Implantat oder einer Osteosynthese montierbar sind.

Der Schritt der Fertigung von Teilelementen kann auch das Anpassen von vorgefertigten Elementen umfassen, beispielsweise ein Abschleifen einer Vorform anhand einer patientenspezifischen Fertigungsvorgabe.

Losgelöst vom anschließenden chirurgischen Eingriff zum Einführen geht es in dem erfindungsgemäße Verfahren um das Bereitstellen von Teilelementen, welche zu einem auf den Patienten abgestimmten Implantat oder einer entsprechenden Osteosynthese zusammenfügbar sind.

Durch die Mehrteiligkeit des Implantats oder der Osteosynthese bzw. Verbindung kann der chirurgische Eingriff minimalinvasiv erfolgen und durch die patientenspezifische Anpassung sind der Platzbedarf und die Funktionalität des Implantats besonders optimiert.

Das Erstellen von Fertigungsvorgaben für zumindest zwei oder mehr zu einem Implantat und/oder zu einer Osteosynthese zusammensetzbaren Teilelementen erfolgt auf Grundlage des erstellten Modells, wobei die Fertigungsvorgaben folgende weitere Angaben umfassen:
C2) eine Materialauswahl bezüglich eines oder mehrerer Materialen für ein jeweiliges Teilelement;
   Dies ermöglicht eine optimale Abstimmung bezüglich einer erwünschten Flexibilität oder Starrheit der Teilelemente.
C3) eine Auswahl einer oder mehrerer Verbindungarten zwischen den jeweiligen Teilelementen.

Dadurch kann eine optimale Verbindung für jedes Teilelement je nach Körperbereich erreicht werden. Dabei kann auf die Verbindung je nach Körperbereich ein hohes Flächengewicht oder erhöhte Zugbelastung wirken. Entsprechend kann die Verbindung gewählt werden.

Als Verbindungsart im Rahmen der vorliegenden Erfindung ist nicht die Auswahl von artgleichen Verbindungsmitteln unterschiedlicher Dimensionierung zu verstehen, sondern die Bereiche der Kraftübertragung zwischen den Verbindungsmitteln unterscheiden sich je nach Verbindungsart. So kann eine Verbindungsart breitflächige Hinterschneidungen, z.B. in Form von Schwalbenschwanzverbindungen, aufweisen, wohingegen andere Verbindungsarten Gewinde aufweisen. Weitere vorteilhafte Ausgestaltungen der Erfindung sind Gegenstand der Unteransprüche.

Diese Angaben verbessern die Anwendungsperformance des Implantats oder der Osteosynthese bzw. der Verbindung.

Die Teilelemente können in vorteilhafter Weise die Teilelemente formschlüssig miteinander verbindbar sein oder zumindest bereichsweise in einem vordefinierten Abstand voneinander beabstandet sind. Ersteres ist besonders bevorzugt um eine feste Anbindung und einen breitflächigen Oberflächenkontakt zu ermöglichen. Der Abstand kann hingegen für eine Verankerung von Gewebe dienen, welche die Verbindung zusätzlich stützt.

Die Fertigungsvorgaben können zudem eine Auswahl von einem oder mehreren Sensoren, Aktuatoren und/oder Wirkstoffen für zumindest eines der Teilelemente umfassen. Dadurch ermöglicht das Implantat eine zusätzliche Überwachung des Gesundheitszustandes des Patienten.

Zum Betrieb und/oder zur Auswertung der Sensorsignale und/oder zur Regelung oder zum Ansteuern der Aktuatoren kann bei der Fertigung der Teilelemente eine Integration und/oder Fixierung einer Stromversorgungsquelle und/oder einer Steuer- und/oder Auswerteeinheit an oder in einem der Teilelemente erfolgen.

Es kann zudem vorteilhaft bei der Fertigung einer Sendeeinheit an oder in dem Implantat oder der Osteosynthese angeordnet werden. Die Sendeeinheit kann eine aktive oder passive Sendeeinheit sein.

Das Erstellen des Modells kann vorteilhaft durch Erstellen eines dreidimensionalen Computer-Modells, vorzugsweise nach der Finite Elemente Methode, erfolgen.

Das Erfassen der Daten gemäß Schritt A) erfolgt anhand eines CT-Scans des individuellen Patienten.

Anhand des CT-Scans wird ein Modell für die individuelle Implantatplanung erstellt, wobei anhand des CT-Scans die Geometrie des Modells eines Implantats einschließlich örtlich variierender individueller Materialstärken und individuell-verteilter Materialarten erfasst wird. Durch den CT-Scan kann beispielsweise die mechanische Belastbarkeit jedes eingescannten Bereichs, beispielsweise gegenüber Druck- Zug- oder Querkräfte bestimmt werden, sowie die variierende Knochendickenverteilung in dem jeweiligen gescannten Bereich.

Auch eine Differenzierung zwischen Haut und Knochen kann vorgenommen werden, so dass bei der Implantatplanung Teilelemente mit Gewebeanteilen berücksichtigt werden können.

Vorzugsweise anhand des CT-Scans kann sodann vorteilhaft eine Erstellung des Modells die unterschiedlichen Materialparameter und Materialgrenzen, insbesondere die Materialdicke, die Materialstreckgrenze, die Materialbruchgrenze, insbesondere bei mechanischer Belastung von unterschiedlichen Richtungen, und/oder die Materialelastizität von Teilbereichen des Implantats berücksichtigt werden

Die Auswahl der Art der Verbindung einzelner Teilelemente erfolgt sodann in Abhängigkeit von individuellen Lastfällen auf das Teilelement anhand des Modells. Dabei stehen mehrere Verbindungstypen zur Verfügung, welche eine unterschiedliche mechanische Verbindung der Teilelemente je nach Lastfall, z.B. in Abhängigkeit von Querkräften auf die Verbindung, erlauben. So nimmt ein Schwalbenschwanz-Verbindung eine sehr breitflächige Verbindung ein und kann auch bei Querkräften quer jedoch nicht senkrecht zur Verbindungsrichtung eine hohe Widerstandskraft aufweisen. Entsprechend ist der Begriff der Querkräfte im Rahmen der vorliegenden Erfindung auch nachfolgend zu verstehen. Demgegenüber weist eine Steckverbindung durch Anschlussstifte eine geringe Widerstandskraft gegenüber Querkräften auf, was zu einem Abbrechen der Stiftverbindung führen kann.

Anders als im Stand der Technik sind die Teilelemente zumindest im Bereich der Außenkontur des Implantats oder der Osteosynthese individuell angepasst. Daher sind vorteilhaft zumindest 66% aller Teilelemente unterschiedlich zueinander dimensioniert gefertigt sind und besonders bevorzugt zumindest 80% aller Teilelemente unterschiedlich zueinander dimensioniert gefertigt sind. Beim Stand der Technik sind bei mehrteiligen Implantaten zumeist nur zwei Typen von Teilelementen vorgesehen, also sind 50% aller Teilelemente identisch dimensioniert. Dies ermöglicht eine besonders kleinteilige und individualisierte Anpassung.

Die Teilelemente im zusammengesetzten Zustand eines Implantats und/oder einer Osteosynthese definieren vorteilhaft ein Kantenmodell mit einem größeren Volumen als jedes einzelne Teilelement, vorzugsweise ein dreifach größeres Kantenmodell.

Die Teilelemente im zusammengesetzten Zustand des Implantats und/oder der Osteosynthese können vorteilhaft ein Kantenmodell mit einem zumindest 8-fach größeren Volumen definieren als jedes einzelne Teilelement. Die erhöhte Anzahl an Teilelementen ermöglicht eine bessere und patientenindividuellere Konzeptplanung der Materialanforderungen in bestimmten Teilbereichen des Implantats. Großteilige Teilelemente mit einer Vielzahl unterschiedlicher Materialien z.B. für Knochen, künstliche Gewebeteile und/oder künstliche Muskeln sind somit nicht aufwendig auf einem großen Teilelement angeordnet sondern gerade konstruktiv schwierige Abschnitte können aufgrund der Unterteilung in mehr Teilelemente leichter vorgefertigt werden.

Die Teilelemente weisen vorzugsweise aufgrund ihrer unterschiedlichen Funktionalität unterschiedliche Materialien auf. So ist in einer vorteilhaften Ausführungsvariante ein erstes Teilelement der Teilelemente aus einem ersten Material und/oder einer ersten Materialkombination gefertigt und ein zweites Teilelement der Teilelemente aus einem zweiten Material und/oder einer zweiten Materialkombination gefertigt. Eine erste Materialkombination kann z.B. Gummi und Titan als Verbundelement sein und eine zweite Materialkombination PLA und Titan oder auch nur reines Titan.

Die auf das Volumen gemittelte Dichte und/oder das Volumen gemittelte Elastizitätsmodul des ersten Materials und/oder der ersten Materialkombination ist dabei größer als die auf das Volumen gemittelte Dichte und/oder das auf das Volumen gemittelte Elastizitätsmodul der zweiten Materialkombination. Das heißt man ermittelt die Dichte und/oder das E-Modul des Gummis und multipliziert diesen Wert mit dem prozentualen Volumenanteil dieses Materials im Teilelement. Sodann ermittelt man die Dichte und/oder das E-Modul des Titans und multipliziert diesen Wert mit dem prozentualen Volumenanteil des Titans im Teilelement. Die beiden Werte werden dann addiert und auf hundert Prozent normiert und können dann mit dem entsprechenden Wert des Materials oder der Materialkombination des zweiten Teilelements verglichen werden. Die erste und/oder zweite Materialkombination braucht sich dabei auch nur hinsichtlich des Anteils der Einzelkomponenten in der Materialkombination zu unterscheiden, z.B. hinsichtlich des Anteils an Gummi oder Titan. Das E-Modul und/oder die Dichte und/oder das Volumen der Materialkomponenten kann unter Standardbedingungen mit im Fachgebiet üblichen Messgeräten unter Berücksichtigung der üblichen Schwankungsbreiten ermittelt werden. Der Nachweis des Volumens kann durch Abmessung, ggf. unter Zuhilfenahme einer laserunterstützten Scanmethode, bestimmt werden. Die Dichte der Implantate oder einzelner Teilelemente kann durch radiologische Bestimmung z.B. mittels CT, beispielsweise durch das Gerät Somatom Flash von Siemens ermittelt werden. Die Elastizität der Teilelemente des Implantats oder der Osteosynthese, insbesondere auch der Gewebeanteile kann mittels des Messgeräts Bioindenter an der CSEM nachgewiesen werden. Durch die unterschiedlichen Dichten und E-Module können die Teilelemente sehr individuell auf die Situation des Patienten angepasst werden.

Auf Basis des CT-Scans des individuellen Patienten wird ein Modell - vornehmlich der knöchernen Strukturen - des Kopfes für die individuelle Implantatplanung erstellt. Dieses Modell wird in einem weiteren Schritt in ein korrespondierendes FE-Modell (FE = Finite Elemente) angepasst in dem die unterschiedlichen Gewebestrukturen wie auch das Implantat unter biomechanischen Gesichtspunkten betrachtet und simuliert werden können. Dafür wird die Geometrie der Strukturen/Implantat volumetrisch vernetzt. Dabei wird bei der Vernetzung die Unterteilung des Implantates und die unterschiedlichen Materialparameter und Materialgrenzen berücksichtigt. Anhand dieser Vorbereitungsschritte können Aussagen bezüglich der Stabilität/Materialgrenzen des Implantates und der Verbindungstypen basierend auf individuellen Lastfällen mittels Simulation getroffen werden.

Dabei ist es insbesondere von Vorteil, wenn ein erstes Teilelement der Teilelemente aus einem ersten Material und/oder einer ersten Materialkombination, z.B. einem Verbundmaterial, gefertigt wird und ein zweites Teilelement der Teilelemente aus einem zweiten Material und/oder einer zweiten Materialkombination gefertigt wird, wobei die auf das Volumen gemittelte Dichte und/oder das Volumen gemittelte Elastizitätsmodul des ersten Materials und/oder der ersten Materialkombination größer ist, vorzugsweise zumindest 1,2-fach größer ist, besonders bevorzugt zumindest 1,5-fach größer ist als die auf das Volumen gemittelte Dichte und/oder das auf das Volumen gemittelte Elastizitätsmodul des zweiten Materials und/oder der zweiten Materialkombination.

Alternativ oder zusätzlich kann ein Teilelement aus einem ersten Material und aus einem zweiten Material gefertigt sein, wobei die Dichte und/oder das Elastizitätsmodul des ersten Materials vorteilhaft größer ist, vorzugsweise zumindest 1,2-fach größer ist, besonders bevorzugt zumindest 1,5-fach größer ist als die Dichte und/oder das Elastizitätsmodul des zweiten Materials.

Der oder die Aktuatoren und/oder der oder die Sensoren eines oder mehrerer Teilelemente sind vorzugsweise und vorteilhaft in MEMS-Bauweise ausgebildet und die Sensorelemente und/oder Aktuatorelemente, ohne Auswerteeinheit und/oder Stromversorgungseinheit, sind besonders bevorzugt kleiner als 1 mm³.

Eines oder mehrere Teilelemente können vorteilhaft aus einem resorbierbaren Material und besonders bevorzugt aus einem resorbierbaren Kunststoff oder Magnesium bestehen. Diese ermöglichen einen unterstützenden jedoch nicht dauerhaften Aufbau von Körperteilen.

Für eine einfache Montage ist es von Vorteil, wenn die Verbindungen der Teilelemente als Steckverbindungen ausgestaltet sind. Diese Steckverbindungen können besonders bevorzugt über einen zusätzlichen Einrast-mechanismus verfügen, welche die Teilelemente nach dem Zusammenstecken miteinander verrastet. Alternativ und ebenfalls vorteilhaft können die Teilelemente über ein Filmscharnier miteinander verbunden sein oder eine Klebung.

Weiterhin erfindungsgemäß ist ein mehrteiliges Implantat und/oder mehrteilige Osteosynthese, welches oder welche hergestellt wird nach einem erfindungsgemäßen Verfahren.

Durch das Implantat oder die Osteosynthese kann vorteilhaft ein körpereigenes Element bereichsweise oder vollständig ersetzt werden. Hierfür weist das Implantat eine Anschlussfläche zum Ansatz an eine vorgefertigte Schnittfläche auf. Diese Schnittfläche kann durch einen vorbereitenden jedoch nicht erfindungsgemäßen Schritt des Abtrennens eines Bruchsegments im Körper bereitgestellt werden. Das Implantat oder die Osteosynthese dient somit in dieser bevorzugten Ausführungsvariante nicht einer Knochenstabilisation sondern als dessen Ersatz.

Die vorliegende Erfindung wird anhand mehrerer Ausführungsbeispiele und unter Zuhilfenahme der beiliegenden Figuren näher erläutert. Es zeigt:
- Fig. 1: schematische Darstellung einer ersten Verbindungsvariante zwischen Teilelementen eines Implantats oder einer Osteosynthese;
- Fig. 2: schematische Darstellung einer zweiten Verbindungsvariante zwischen Teilelementen eines Implantats oder einer Osteosynthese;
- Fig. 3: schematische Darstellung einer dritten Verbindungsvariante zwischen Teilelementen eines Implantats oder einer Osteosynthese;
- Fig. 4: schematische Darstellung einer vierten Verbindungsvariante zwischen Teilelementen eines Implantats oder einer Osteosynthese; und
- Fig. 5: Ablaufdiagramm eines erfindungsgemäßen Verfahrens zur Bereitstellung eines mehrteiligen Implantats oder einer mehrteiligen Osteosynthese.

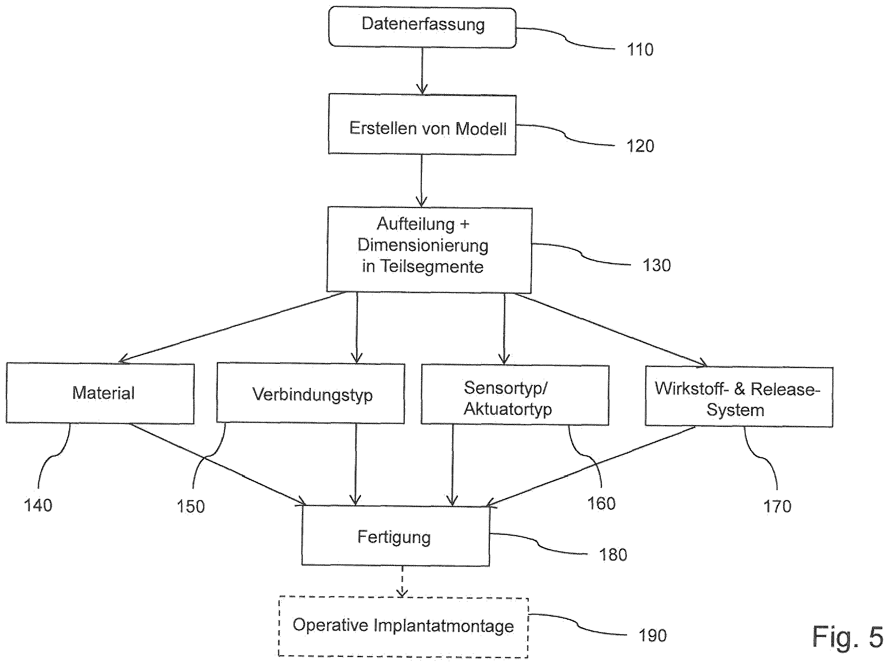

Fig. 5 zeigt einen Verfahrensablauf eines erfindungsgemäßen Verfahrens zur Bereitstellung eines mehrteiligen Implantats oder einer mehrteiligen Osteosynthese.

In einem ersten Schritt erfolgt eine Datenerfassung 110. Bei dieser Datenerfassung handelt es sich um eine individuelle Datenerfassung eines Patienten. Es handelt sich somit um individuelle Patientendaten, welche auf Basis eines CT-Scans erstellt wurden.

Zusätzlich zur Erfassung von dreidimensionalen Aufnahmen, kann auch ein Erfassen weiterer chemischer und/oder physikalischer Daten erfolgen. Dies kann beispielsweise das Erfassen der Konzentration spezifischer Verbindungen im Körper, z.B. einer lonenkonzentration und/oder des pH-Werts, der Körpertemperatur, des Herzschlags und/oder des Kontraktions- und Expansionsvolumens einzelner Organe umfassen. Diese Werte sind teilweise ebenfalls patientenspezifisch.

Auf Basis dieser Datenerfassung kann ein Modell 120 eines betroffenen Organs und/oder eines betroffenen Knochens und/oder der Umgebung des Organs und/oder Knochens erstellt werden. Handelt es sich bei dem Modell um die Umgebung, so kann aufgrund dieses Modells ein Raumbedarf des benötigten Implantats und/oder der benötigten Osteosynthese ermittelt werden.

Bei dem Modell 120 kann es sich um ein digitales dreidimensionales ComputerModell handeln oder um ein reales dreidimensionales Computermodell, welches vorzugsweise durch Stereolithographie, besonders bevorzugt durch Rapid-Prototyping, und insbesondere durch ein generatives Fertigungsverfahren, wie z.B. selektives Laserschmelzen oder durch einen 3D-Druck hergestellt wurde.

Anhand dieses Modells 120 werden vor der Ausgestaltung des Implantats und/oder der Osteosynthese Fertigungsvorgaben erstellt, dies umfasst u. a. eine Aufteilung und Dimensionierung 130 von Einzelteilen bzw. Teilelementen des dreidimensionalen individualisierten Implantats oder der dreidimensionalen individualisierten Osteosynthese.

Das Modell 120 und die Teilelemente des Modells können zur Definition ihrer dreidimensionalen Ausbreitung jeweils durch ein imaginäres quaderförmiges Kantenmodell begrenzt werden, wobei diese Kantenmodelle die maximale Ausdehnung des Modells 120 und die maximale Ausdehnung der Teilelemente des Modells aufweisen. Somit ist jedes der Teilelemente kleiner als das Gesamtkonstrukt des Implantats und/oder der Osteosynthese.

Dabei ist das Volumen des Kantenmodells, welches das Modell 120 des Implantats oder der Osteosynthese begrenzt, größer als das Volumen des Kantenmodells, welches ein Teilelement des Modells 120 begrenzt.

Vorzugsweise ist das Volumen des Kantenmodells des Modells 120 zumindest dreimal größer, besonders bevorzugt zumindest 8-fach größer, als das Volumen des Kantenmodells des Teilelements des Modells 120.

Besonders bevorzugt weist zumindest die Querschnittsfläche eines Teilelements einen geringeren Flächeninhalt auf als die Querschnittsfläche des Implantats oder der Osteosynthese welche die Querschnittsfläche des Teilelements beinhaltet. Das heißt der Querschnitt des Implantats oder der Osteosynthese auf Höhe des Querschnitts des Teilelements ist größer als der des Teilelements, vorzugsweise zumindest doppelt so groß, besonders bevorzugt zumindest 4-mal so groß.

Die Definition gemäß Kantenmodell bezieht sich dabei auf ein Modell des Implantats und/oder der Osteosynthese. Handelt es sich bei dem Modell um ein Modell der Umgebung, so ist anhand des Modells der dreidimensionale Raumbedarf des benötigten Implantats oder der benötigten Osteosynthese zu erstellen und das Kantenmodell ist auf Grundlage dieses Raumbedarfs zu erstellen.

Durch das Modell 120 wird das Makrodesign des bereitzustellenden Implantats oder der bereitzustellenden Osteosynthese abgestimmt, welches Implantat und/oder welche Osteosynthese somit individuell und optimal in allen drei Raumrichtungen auf den Patienten angepasst sind. Dabei ändern sich typischerweise die Abmessungen des Modells und des nach dem Vorgaben des Modells modellierten Implantats oder der Osteosynthese für jeden Patienten in allen drei Raumrichtungen.

Nach der Aufteilung des Modells in Teilelemente 130 erfolgt eine segmentspezifische Auswahl zur Ausgestaltung hinsichtlich eines jeweiligen Teilelements.

Diese Auswahl betrifft u. a. die Auswahl des Materials 140 eines jeweiligen Teilelements und die Auswahl der Verbindung 150 der Teilelemente untereinander.

Die Materialauswahl 140 kann besonders bevorzugt eine oder mehrere der folgenden Materialien umfassen: Formgedächtnis-Material, Self-Assembly-Material, Gradienten-Material, künstliche Muskeln und/oder Gewebeanteile, vorzugsweise Haut, Schleimhaut, Knochenhaut und/oder Weichgewebe.

Formgedächtnismaterialien können vorzugsweise aus Kunststoff oder einer Legierung, einem sogenannten shape-memory-alloy, bestehen.

Selbstassemblierung (Self-Assembly-Materialien) sind beispielsweise Materialien, die autonom, also ohne äußerliche Einwirkungen eine spezifische Struktur- und Musterbildung zeigen. Zudem können sie aber auch unter Anregung, wie z.B. Licht, mechanische Anregung oder elektrische Anregung, eine neue Form annehmen. Dies kann z.B. eine Struktur mit starren Teilbereichen sein, welche mit Filmscharnieren verbunden ist und welche minimal-invasiv in eine Wunde einführbar und anschließend entfaltbar ist. Das Entfalten kann bevorzugt zu einer vordefinierten Form erfolgen.

Gradienten-Materialien werden aktuell vom Fraunhofer Institut untersucht und entwickelt. Ein Gradienten-Material ist beispielsweise PCU, Polycarbonaturethan.

Künstliche Muskeln werden derzeit u.a. unter dem Projekt "Bionicum" in verschiedenen Forschungsinstituten untersucht. Derartige Muskeln kommen für die Materialauswahl der Teilelemente ebenfalls in Betracht.

Weiterhin ist auch bekannt Gewebe, insbesondere menschliches Gewebe, zu züchten. Dieses Gewebe kann ebenfalls als Material für die Teilelemente eingesetzt werden. Zu dem Gewebe sind u.a. Haut, Schleimhaut, Knochenhaut und Weichgewebe gezählt werden. Alternativ zur Züchtung kann das Gewebe auch dem Patienten oder einem Spender, menschlich oder tierisch, entnommen werden und bei der Materialauswahl berücksichtigt werden.

Die Auswahl der Verbindungsarten kann vorteilhaft derart erfolgen, dass bei der Implantatplanung z.B. durch ein Computerprogramm zumindest die Auswahl zwischen den Verbindungstypen einer Schwalbenschwanzverbindung, einer Stiftsteckverbindung und/oder einer Gelenkverbindung, insbesondere einer Kugelgelenkkopfverbindung, ausgewählt wird. Zumindest diese drei Verbindungstypen stehen bei der Planung als Datensatz zur Verfügung. Als Stiftsteckverbindung im Rahmen der vorliegenden Verbindung sind insbesondere Stiftsteckverbindungen zu verstehen, bei welchen der Stift einen Stiftkopf aufweist, welcher als Kugel oder anderweitiger Vorsprung gegenüber dem Stiftschaft an endständiger Position des Stiftes hervorsteht.

Darüber hinaus sind weitere bevorzugte Materialien für die Materialauswahl Materialien mit Fest-Flüssig oder Fest-Fest - Phasenumwandlungen unter Wärmeeinfluss im Temperaturbereich zwischen 0 bis 85°C oder Lichteinfluss, Graphene-Materialien, magnetische Materialien, Materialkombinationen mit unterschiedlicher Röntgenopazität, thermisch-leitende oder elektrisch-leitende Materialien.

Bei der Materialauswahl des Basismaterials können sowohl Metalle/Legierungen als auch keramische Materialien und/oder Kunststoffe genutzt werden. In einer bevorzugten Ausführungsvariante der Erfindung können insbesondere faserverstärkte Kunststoffe für die Herstellung der Teilelemente genutzt werden, wie z.B. karbonverstärktes Polyetheretherketon (PEEK). Es sind auch Materialkombinationen und Materialverbundstoffe möglich, welche in einem Teilelement vorkommen können. Je nach Beanspruchung des Implantats und/oder der Osteosynthese kann es darauf ankommen, dass ein erstes Teilelement besonders flexibel ausgebildet ist und ein zweites Teilelement des gleichen Implantats oder der gleichen Osteosynthese besonders flexibel, also biegsam und/oder dehnbar und/oder schaumartig, ist. Dies kann durch die Materialauswahl vorab bestimmt und angepasst sein. Jeder Patient hat dabei andere Körpermaße und muskuläre Ausprägungen, sodass die Dimensionierung des Implantats oder der Osteosynthese anhand des vorher erstellten Modells individuell vorgenommen werden kann.

Besonders bevorzugt verwendete Metalle sind Stahl und/oder Titan.

Zu den bevorzugten keramischen Werkstoffen zählen insbesondere auch Werkstoffe aus Magnesium.

Besonders bevorzugt verwendete Kunststoffe sind resorbierbare Kunststoffe vorzugsweise basierend auf Polymilchsäure und/oder deren Derivate, z.B. PLA. Die Kunststoffe können bevorzugt zumindest bereichsweise faserverstärkt sein.

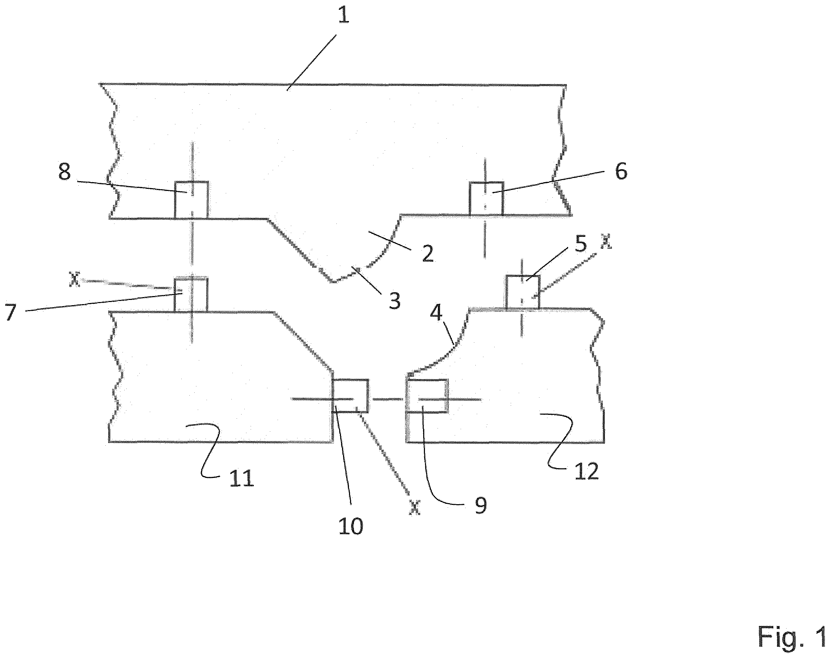

Gleiches gilt für die Auswahl des Typs der Verbindungen zwischen den Teilelementen. Die Verbindung kann teilweise flexibel sein. Dies kann über Kugelgelenke erreicht werden, wie sie z.B. in der US 6 060 641 A offenbart sind. Sie kann allerdings auch starr sein. Verschiedene Verbindungstypen sind in den Fig. 1-4 näher dargestellt. Die vorliegende Erfindung unterscheidet zwischen Verbindungsmitteln, welche Teilelemente des Implantats oder der Osteosynthese miteinander verbinden und zwischen Fixationsmitteln welche das Implantat oder die Osteosynthese an einer bestehenden Struktur z. B. einem Knochengerüst im Körper fixieren. Die Verbindungsmittel können allerdings zugleich auch als Fixationsmittel dienen

Optional kann auch eine Auswahl hinsichtlich von Sensoren und/oder Aktuatoren 160 erfolgen, welche in dem jeweiligen Teilelement integriert sein können. Sensoren können beispielsweise die Körpertemperatur oder den Herzschlag erfassen und an einen Aktuator in Form eines Herzschrittmachers weitergeben, welcher elektrische Impulse zur Stimulation des Herzmuskels aussendet.

Aktuatoren sind dabei Elemente welche elektrische Signale in mechanische oder andere physikalische Größen, z. B. Wärme oder Licht, umsetzen. Dies kann z.B. ein Heizelement sein, welches die Temperatur in einem spezifischen Bereich des Körpers reguliert. Auch Chemostate sind im Rahmen der vorliegenden Erfindung als Aktuatoren zu verstehen. Sie dienen vorzugsweise zur Regulierung eines pH-Wertes, einer lonen- und/oder Sauerstoffkonzentration. Die Aktuatoren können in mikromechanischer bzw. mikroelektromechanischer Bauweise ausgeführt sein.

Sensoren, welche im Rahmen der vorliegenden Erfindung in Teilelementen von Implantaten und/oder Osteosynthesen eingesetzt werden können, können z. B. chemische, elektrische, elektrochemische und/oder physikalische Sensoren zum Erfassen einer patientenspezifischen Größe sein. Bevorzugt eingesetzte Sensoren sind unter dem Begriff "BioMEMS" bekannt. Dabei handelt es sich um mikroelektromechanische Sensoren für den Anwendungsbereich der Diagnostik und der Biotechnologie. Sie umfassen vorzugsweise elektrochemische Sensoren.

Entsprechende Sensoren in mikromechanischer Bauweise als MEMS-Elemente, welche über Festphasen-Elektroden als Referenzelektrode verfügen, können in einem Teilelement eines Implantats oder einer Osteosynthese integriert werden.

Es sind auch Durchflusssensoren bekannt, welche als mikroelektromechanische Sensoren ausgebildet sind. Diese können ebenfalls beispielsweise zur Überwachung einer lokalen Blutzirkulation in einem Teilsegment des Implantat und/oder der Osteosynthese integriert sein.

Es sind zudem Optische Sensoren, Piezoresistive Sensoren, Drucksensoren und/oder Temperatursensoren bekannt, welche vorzugsweise in mikromechanischer Bauweise ausgeführt sein können und welche im Rahmen der vorliegenden Erfindung in einem Teilelement eines Implantats und/oder einer Osteosynthese integriert sein können.

Weiterhin kann das Implantat oder die Osteosynthese auch eine Stromversorgungsquelle aufweisen zur Energieversorgung der vorgenannten Aktuatoren und/oder Sensoren.

Weiterhin kann das Implantat oder die Osteosynthese eine Auswerteeinheit aufweisen, welche die von einem oder mehreren Sensoren ermittelten Daten erfasst und ggf. auswertet oder an eine externe Recheneinheit, also eine Recheneinheit außerhalb des Körpers, zur weiteren Auswertung, vorzugsweise durch Wireless-Datenübertragung, übermittelt. Zudem kann die Steuereinheit aufgrund der ermittelten Daten einen oder mehrere der vorgenannten Aktuatoren ansteuern. In einer bevorzugten Variante kann die Steuer- und die Auswerteeinheit als ein Bauteil realisiert sein. Die Steuerung kann sowohl digital als auch analog erfolgen und kann sowohl ein Zusammenwirken von Teilelementen erreichen als auch eine Kommunikation mit Geräten außerhalb des Körpers.

Weiterhin optional kann eine Auswahl von Wirkstoff- und/oder Releasesystemen 170 erfolgen, mit welchen ein Teilelement des Implantats oder der Osteosynthese beschichtet wird oder welche im Material des Teilelements integriert bzw. eingebunden werden.

Unter dem Begriff Wirkstoffe bzw. Wirkstoffsysteme sind Arzneistoffe zu verstehen. Dies können z.B. Antibiotika, Immunsuppressiva, Wachstumshormone oder Zytostatika sein.

Releasesysteme umfassen alle Substanzen, welche typischerweise zusätzlich dem Arzneistoff zugegeben werden, beispielsweise um die Freisetzung des Arzneistoffes im Körper hinsichtlich des Abgabezeitraumes und des Abgabeortes zu steuern oder um den Arzneistoff unter verschiedenen Bedingungen haltbar zu machen. Typische Substanzen sind z.B. Tocopherol, welches oft als Antioxidans zugesetzt wird, oder Polysaccharide, z.B. Chitin, zur Mikroverkapselung eines entsprechenden Arzneistoffes. Die Wahl des Releasesystems hängt u. a. auch von der Wahl des Materials, also des Basismaterials, ab aus welchem das Teilelement hergestellt werden soll.

In einem weiteren Schritt erfolgt die Fertigung 180 der Teilelemente entsprechend der vorgenannten Auswahlkriterien. Die jeweiligen Teilelemente können vorzugsweise durch generative Fertigungsverfahren, z. B. SLM-Verfahren, durch 3D-Druckverfahren und/oder durch mechanische, insbesondere materialabtragende Verfahren, wie z. B. Fräsen, anhand des Modells ausgebildet werden.

Die Teilelemente können durch eine operative Montage 190 zu einem Implantat oder einer Osteosynthese zusammengesetzt werden. Dabei können die Teilelemente des Implantats oder der Osteosynthese im Rahmen eines operativen Eingriffs durch eine Operationswunde in den menschlichen Körper eingeführt und im Körper zum Implantat oder zur Osteosynthese zusammengesetzt bzw. montiert werden. Die Montage kann zudem die Fixierung des Implantats z. B. an einem bestehenden Knochengerüst umfassen.

Das Einführen der Teilelemente des Implantats oder der Osteosynthese ermöglicht eine Verringerung der Dimensionierung einer Operationswunde, sodass das Einsetzen des individuell auf den Patient angepassten Implantats oder der Osteosynthese im Rahmen einer minimalinvasiven Vorgehensweise bei einer Operation erfolgen kann, wodurch die Chancen und der Zeitraum einer erfolgreichen Verheilung und der Regeneration deutlich erhöht wird.

Der operative Eingriff umfassend das Einführen der Teilelemente durch die Operationswunde und deren Zusammensetzung zu einem Implantat oder einer Osteosynthese kann endoskopisch, von Hand, navigiert und/oder roboter-unterstützt erfolgen.

In Fig. 1-4 sind ausschnittsweise einzelne Beispiele für Verbindungen der Teilelemente zu einem Implantat oder einer Osteosynthese dargestellt. Diese Verbindung erfolgt im Körper vorzugsweise im Rahmen einer minimalinvasiven Operation.

In Fig. 1 erkennt man die Teilelemente 1, 11 und 12. Dabei kann es sich beispielsweise um Knochenplatten aus PLA-Kunststoff handeln. Diese weisen ungleichmäßige Strukturen mit Vorsprüngen 2 und Aufnahmen 4 auf, welche auf, deren Kontaktflächen 3 unter einen Formenschluss der Teilelemente 1, 11 und 12 aneinander anliegen.

Die Form der Teilelemente und deren Material können bevorzugt aufgrund einer simulierten Belastungsanalyse erfolgen. Entsprechende Simulationsprogramme sind kommerziell erhältlich und können z.B. als CAD-System im Rahmen der Finiten Elemente-Methode ermittelt werden.

Die Teilelemente 1, 11 und 12 weisen zudem gleichartige Vorsprünge 5, 7, 10 und Aufnahmen 6, 8, 9 auf um eine kraft- und formschlüssige Verbindung in der bevorzugten Form einer Steckverbindung vorzugsweise in mehrere Raumrichtungen zu ermöglichen.

Als Verbindungen zwischen Teilelementen ist auch eine oder mehrere bionische Verbindungen bevorzugt. Diese bionische Verbindung kann vorzugsweise durch körpereigenes Gewebe, z.B. Muskeln, angesteuert werden oder durch Nerventransmission.

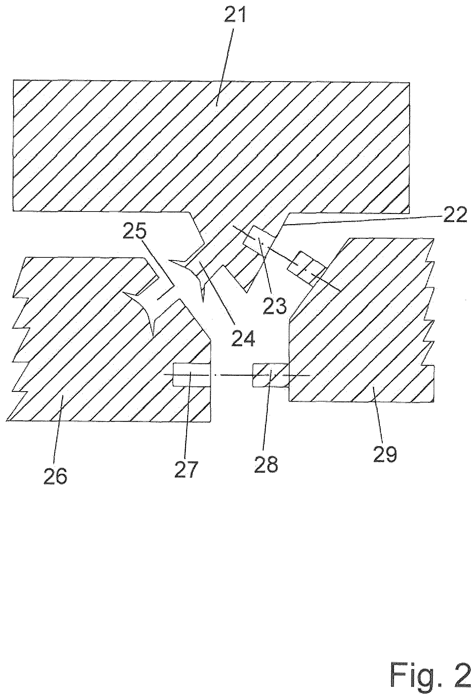

Fig. 2 zeigt unterschiedliche Arten von Steckverbindungen, deren Vorsprünge und Aufnahme an Teilelementen 21, 26, 29 angeordnet sind. Teilelement 29 weist gleichartige Vorsprünge 28 auf, welche in unterschiedlichen Raumrichtungen aus dem Teilelement hervorstehen. Teilelement 26 weist zwei Aufnahmen 25 und 27 auf, welche unterschiedliche geometrische Ausgestaltungen aufweisen. Während eine erste Aufnahme 25 zylindrisch ausgebildet ist, weist die zweite der Aufnahmen 25 einen Hinterschnitt auf. In diese Aufnahme mit Hinterschnitt greift formschlüssig und kraftschlüssig ein Vorsprung 24 des Teilelements 21 ein. Dieses Teilelement 21 weist zudem eine Aufnahme 3 auf in welche der Vorsprung 28 form- und kraftschlüssig einsteckbar ist.

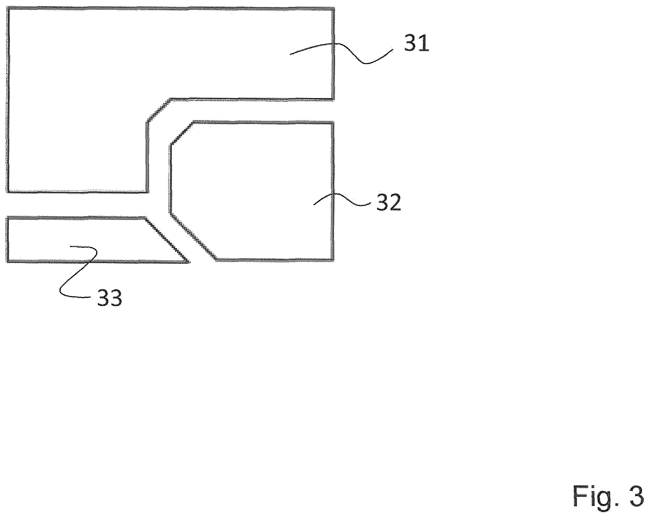

Fig. 3 zeigt drei Teilelemente 31, 32, 33. Diese können zumindest bereichsweise magnetisches Material aufweisen oder zumindest an den Kontaktflächen bereichsweise eine magnetische Beschichtung aufweisen. Weiterhin optional können die Kontaktflächen eine oder mehrere Substanzen aufweisen, welche sich im Körper unter Einwirkung von Körperflüssigkeiten miteinander unter Materialschluss verbinden, sodass allmählich aus den Teilelementen ein einheitlicher Körper gebildet wird. Derartige Substanzen können verkapselte gesundheitlichunbedenkliche Haftvermittler oder Haftvermittlerkomponenten sein, welche in einer bioabbaubaren Verkapselung eingeschlossen sind.

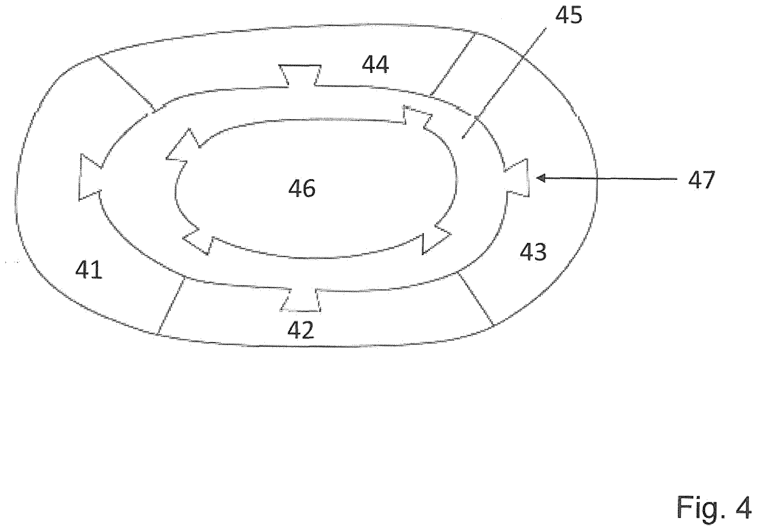

Fig. 4 zeigt die Draufsicht einer plattenförmigen Osteosynthese. Dabei sind die Teilelemente 41, 42, 43, 44, 45, 46 bogenförmig ausgestaltet und über eine sogenannte Schwalbenschwanz-Verbindung 47 miteinander verbunden.

Die Fig. 1-4 stellen nur einige bevorzugte Beispiele für Verbindungen dar. Es sind jedoch auch Bajonettverbindungen oder andere Verbindungsvarianten der Teilelemente im Rahmen der vorliegenden Erfindung realisierbar

In einer bevorzugten Ausführungsvariante der Erfindung kann ein Teilelement oder eine Verbindung eine Sollbruchstelle aufweisen, in welchem das montierte Implantat oder die montierte Osteosynthese bei stärkerer mechanischer Belastung bevorzugt bricht.

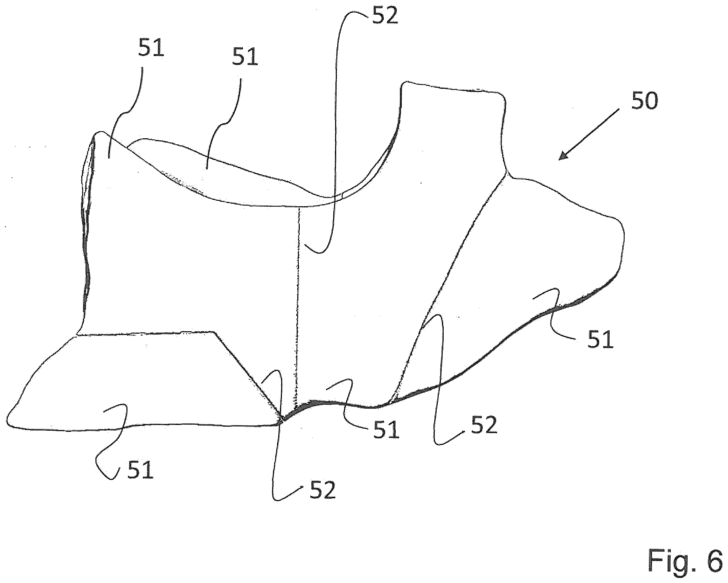

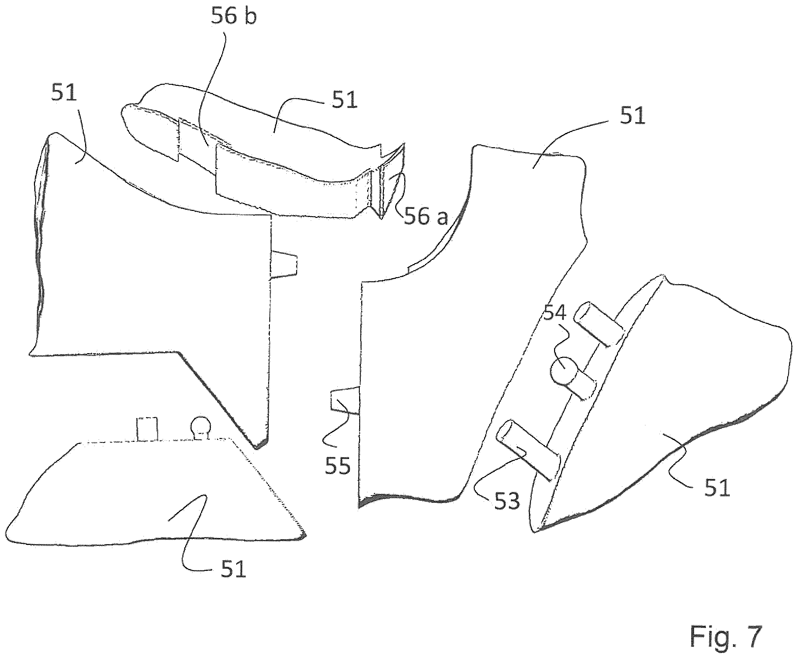

Fig. 6 und 7 zeigen ein Beispiel für ein entsprechendes Implantat 50 für ein Jochbein. In Fig. 6 ist das Implantat 50 aus mehreren Teilelementen 51 zusammengesetzt. Deutlich erkennt man dabei die Übergänge 52 zwischen den Teilelementen. Jedes der Teilelemente ist gegenüber den anderen Teilelementen unterschiedlich dimensioniert.

In Fig. 7 ist das Implantat im auseinandergenommenen Zustand dargestellt. Man erkennt, dass jeder Übergang eine andersartige Verbindung aufweist. Dies kann z.B. eine Steckverbindung mit zylindrischen Steckköpfen 53 sein. Diese ermöglichen eine Stabilität seitlich zur Steckrichtung. Für ein besseren Widerstand gegen ein Auseinanderziehen empfiehlt sich eine Steckverbindung mit Kugelkopf 54. Weine weitere Verbindungsvariante kann eine Verzahnung 55 sein. Schließlich erkennt man, dass ein Teilelement 51 mittels einer Schwalbenschwanz-Steckverbindung 56a und 56b am restlichen Implantat befestigt ist. Auf diesem Teilelement kann z.B. ein Auge aufliegen, welches nur sehr geringes Gewicht auf dieses Teilelement in Steckrichtung ausübt. In der Richtung senkrecht zu den Verbindungsflächen kann die Schwalbenschwanz-Steckverbindung 56a und 56b jedoch sehr starken Zugkräften widerstehen.

Fig. 7 zeigt, dass durch Auswahl der Verbindungstypen eine Kräfteverteilung auf das Implantat nachempfunden werden kann, welche auch im Körper auf das Implantat natürlich auftritt. Entsprechend kann die Festigkeit der Verbindung zwischen den unterschiedlichen Teilelementen 51 gewählt werden.

Die vorgenannten Ausführungsbeispiele beziehen sich auf den menschlichen Körper. Es versteht sich allerdings bereits aus dem Kontext der vorliegenden Erfindung, dass die Erfindung auch bei Tieren eingesetzt werden kann.

## Patentansprüche

1. Verfahren zum Bereitstellen von Teilelementen eines mehrteiligen Implantats oder einer mehrteiligen Osteosynthese vor deren Einführung in einen menschlichen und/oder tierischen Körper,
das die folgenden Schritte umfasst:
A) Erfassen von Daten (110) anhand eines CT-Scans eines Patienten, für welchen das Implantat und/oder die Osteosynthese bestimmt ist;
B) Erstellen eines Modells (120) anhand der erfassten Daten;
C) Erstellen von Fertigungsvorgaben für zumindest zwei oder mehr zu einem Implantat und/oder zu einer Osteosynthese zusammensetzbare Teilelemente auf Grundlage des erstellten Modells, wobei die Fertigungsvorgaben umfassen:
C1) eine Dimensionierung der Teilelemente (130);
und wobei die Fertigungsvorgaben umfassen:
C2) eine Materialauswahl (140) bezüglich eines oder mehrerer Materialen für ein jeweiliges Teilelement; und/oder
C3) eine Auswahl einer oder mehrerer Verbindungarten (150) zwischen den jeweiligen Teilelementen;
D) Fertigung (180) der Teilelemente auf Basis der Fertigungsvorgaben; wobei die Teilelemente miteinander zu einem Implantat oder einer Osteosynthese montierbar sind, **dadurch gekennzeichnet, dass** anhand des CT-Scans ein Modell für die individuelle Implantatplanung oder Osteosyntheseplanung erstellt wird, wobei anhand des CT-Scans die Geometrie des Modells eines Implantats oder einer Osteosynthese einschließlich örtlich variierender individueller Materialstärken und individuell-verteilter Materialarten erfasst wird.

2. Verfahren nach Anspruch 1, **dadurch gekennzeichnet, dass** die Fertigungsvorgaben eine Materialauswahl (140) bezüglich eines oder mehrerer Materialien für ein jeweiliges Teilelement umfassen und die Materialauswahl (140) eines oder mehrere der folgenden Materialien umfasst: Formgedächtnis-Material, Self-Assembly-Material, Gradienten-Material, künstliche Muskeln und/oder Gewebeanteile, vorzugsweise Haut, Schleimhaut, Knochenhaut, Weichgewebe.

3. Verfahren nach einem der vorhergehenden Ansprüche 1 oder 2, **dadurch gekennzeichnet, dass** die Fertigungsvorgaben eine Auswahl einer oder mehrerer Verbindungsarten (150) zwischen den jeweiligen Teilelementen umfassen und
die Auswahl der Verbindungsarten zumindest die Auswahl zwischen den Verbindungstypen einer Schwalbenschwanzverbindung, einer Stiftsteckverbindung und/oder einer Gelenkverbindung, insbesondere einer Kugelgelenkkopfverbindung, umfasst.

4. Verfahren nach einem der vorhergehenden Ansprüche, **dadurch gekennzeichnet, dass das** Erstellen des Modells (120) als Erstellen eines dreidimensionalen Computer-Modells, vorzugsweise nach der Finite Elemente Methode, erfolgt.

5. Verfahren nach einem der vorhergehenden Ansprüche, **dadurch gekennzeichnet, dass** das Erfassen von Daten durch ein bildgebendes Verfahren zum Zwecke des Erstellens eines dreidimensionalen Modells erfolgt.

6. Verfahren nach einem der vorhergehenden Ansprüche, **dadurch gekennzeichnet, dass** zur Erstellung des Modells die unterschiedlichen Materialparameter und Materialgrenzen, insbesondere die Materialdicke, die Materialstreckgrenze, die Materialbruchgrenze, insbesondere bei mechanischer Belastung von unterschiedlichen Richtungen, und/oder die Materialelastizität von Teilbereichen des Implantats berücksichtigt werden

7. Verfahren nach einem der vorhergehenden Ansprüche, **dadurch gekennzeichnet, dass** die Auswahl der Art der Verbindung einzelner Teilelemente in Abhängigkeit von individuellen Lastfällen auf das Teilelement anhand des Modells erfolgt.

8. Verfahren nach einem der vorhergehenden Ansprüche, **dadurch gekennzeichnet, dass** zumindest 66% aller Teilelemente, vorzugsweise zumindest 80% aller Teilelemente, unterschiedlich zueinander dimensioniert gefertigt sind.

9. Verfahren nach einem der vorhergehenden Ansprüche, **dadurch gekennzeichnet, dass** die Teilelemente im zusammengesetzten Zustand eines Implantats und/oder einer Osteosynthese ein Kantenmodell mit einem größeren Volumen definieren als jedes einzelne Teilelement, vorzugsweise ein zumindest dreifach größeres Kantenmodell.

10. Verfahren nach Anspruch 9, **dadurch gekennzeichnet, dass** die Teilelemente im zusammengesetzten Zustand des Implantats und/oder der Osteosynthese ein Kantenmodell mit einem zumindest 8-fach größeren Volumen definieren als jedes einzelne Teilelement.

11. Verfahren nach einem der vorhergehenden Ansprüche, **dadurch gekennzeichnet, dass** ein erstes Teilelement der Teilelemente aus einem ersten Material und/oder einer ersten Materialkombination gefertigt wird und ein zweites Teilelement der Teilelemente aus einem zweiten Material und/oder einer zweiten Materialkombination gefertigt wird, wobei die auf das Volumen gemittelte Dichte und/oder das Volumen gemittelte Elastizitätsmodul des ersten Materials und/oder der ersten Materialkombination größer ist, vorzugsweise zumindest 1,2-fach größer ist, besonders bevorzugt zumindest 1,5-fach größer ist als die auf das Volumen gemittelte Dichte und/oder das auf das Volumen gemittelte Elastizitätsmodul des zweiten Materials und/oder der zweiten Materialkombination.

12. Verfahren nach einem der vorhergehenden Ansprüche, **dadurch gekennzeichnet, dass** ein Teilelement aus einem ersten Material und aus einem zweiten Material gefertigt ist, wobei die Dichte und/oder das Elastizitätsmodul des ersten Materials größer ist, vorzugsweise zumindest 1,2-fach größer ist, besonders bevorzugt zumindest 1,5-fach größer ist als die Dichte und/oder das Elastizitätsmodul des zweiten Materials.

13. Verfahren nach einem der vorhergehenden Ansprüche, **dadurch gekennzeichnet, dass** eines oder mehrere Teilelemente aus einem resorbierbaren Werkstoff, vorzugsweise aus einem resorbierbaren Kunststoff oder Magnesium, bestehen.

## Claims

1. Method for providing sub-elements of a multipart implant or a multipart osteosynthesis prior to their introduction into a human and/or animal body, comprising the following steps:
A) acquiring data (110) of a patient for whom the implant and/or osteosynthesis is intended by means of a CT scan;
B) creating a model (120) based on the collected data;
C) creating manufacturing specifications for at least two or more sub-elements which can be assembled to form an implant and/or an osteosynthesis on the basis of the model created, wherein the manufacturing specifications comprise:
C1) a dimensioning of the sub-elements (130);
and wherein the manufacturing specifications comprise:
C2) a material selection (140) relating to one or more materials for a respective sub-element; and/or
C3) a selection of one or more connection types (150) between the respective sub-elements;
D) manufacturing (180) of the sub-elements on the basis of the manufacturing specifications; wherein the sub-elements can be assembled with one another to form an implant or an osteosynthesis, **characterized in that** a model for individual implant planning or osteosynthesis planning is created on the basis of the CT scan, wherein the geometry of the model of an implant or an osteosynthesis, including locally varying individual material thicknesses and individually distributed material types, is recorded on the basis of the CT scan.

2. Method according to claim 1, **characterized in that** the manufacturing specifications comprise a material selection (140) with respect to one or more materials for a respective sub-element and the material selection (140) comprises one or more of the following materials: shape memory material, self-assembly material, gradient material, artificial muscles and/or tissue portions, preferably skin, mucosa, periosteum, soft tissue.

3. Method according to one of the preceding claims 1 or 2, **characterized in that** the manufacturing specifications comprise a selection of one or more connection types (150) between the respective sub-elements and the selection of the connection types comprises at least the selection between the connection types of a dovetail connection, a pin plug-in connection and/or an articulated connection, in particular a ball joint head connection.

4. Method according to one of the preceding claims, **characterized in that the** creation of the model (120) is carried out as the creation of a three-dimensional computer model, preferably according to the finite element method.

5. Method according to one of the preceding claims, **characterized in that** the acquisition of data is carried out by an imaging method for the purpose of creating a three-dimensional model.

6. Method according to one of the preceding claims, **characterized in that,** in order to create the model, the different material parameters and material limits, in particular the material thickness, the material yield strength, the material fracture limit, in particular under mechanical loading from different directions, and/or the material elasticity of subregions of the implant are taken into account.

7. Method according to one of the preceding claims, **characterized in that** the selection of the type of connection of individual sub-elements is carried out depending on individual load cases on the sub-element on the basis of the model.

8. Method according to one of the preceding claims, **characterized in that** at least 66% of all sub-elements, preferably at least 80% of all sub-elements, are manufactured in a differently dimensioned manner to each other.

9. Method according to one of the preceding claims, **characterized in that** the sub-elements in the assembled state of an implant and/or an osteosynthesis define an edge model with a greater volume than each individual sub-element, preferably an edge model at least three times greater.

10. Method according to claim 9, **characterized in that** the sub-elements in the assembled state of the implant and/or the osteosynthesis define an edge model with a volume at least 8 times greater than each individual sub-element.

11. Method according to one of the preceding claims, **characterized in that** a first sub-element of the sub-elements is made of a first material and/or a first material combination and a second sub-element of the sub-elements is made of a second material and/or a second material combination, wherein the density and/or elasticity modulus averaged to the volume of the first material and/or the first material combination is greater, preferably at least 1.2 times greater, particularly preferably at least 1.5 times greater, than the density and/or the elasticity modulus averaged to the volume of the second material and/or the second material combination.

12. Method according to one of the preceding claims, **characterized in that** a sub-element is made of a first material and of a second material, wherein the density and/or the elasticity modulus of the first material is greater, preferably at least 1.2 times greater, particularly preferably at least 1.5 times greater, than the density and/or the elasticity modulus of the second material.

13. Method according to one of the preceding claims, **characterized in that** one or more sub-elements consist of an absorbable material, preferably of an absorbable plastic or magnesium.

## Revendications

1. Procédé pour mettre à disposition des éléments partiels d'un implant en plusieurs parties ou d'une ostéosynthèse en plusieurs parties avant leur introduction dans un corps humain et/ou animal,
qui comprend les étapes suivantes :
A) acquisition de données (110) à l'aide d'un balayage de tomographie d'un patient auquel l'implant et/ou l'ostéosynthèse est destiné(e) ;
B) élaboration d'un modèle (120) à l'aide des données acquises ;
C) élaboration de spécifications de fabrication pour au moins deux ou plusieurs éléments partiels pouvant être assemblés pour former un implant et/ou une ostéosynthèse sur la base du modèle élaboré, les spécifications de fabrication comprenant :
C1) un dimensionnement des éléments partiels (130) et
C2) une sélection de matériaux (140) concernant un ou plusieurs matériaux pour un élément partiel respectif ; et/ou
C3) une sélection d'un ou plusieurs types d'assemblage (150) entre les éléments partiels respectifs ;
D) fabrication (180) des éléments partiels sur la base des spécifications de fabrication, les éléments partiels pouvant être montés ensemble pour former un implant ou une ostéosynthèse, **caractérisé en ce qu'**un modèle pour la planification individuelle de l'implant ou de l'ostéosynthèse est élaboré à l'aide du balayage de tomographie, la géométrie du modèle d'un implant ou d'une ostéosynthèse, y compris les épaisseurs de matériau individuelles variant localement et les types de matériau répartis individuellement, étant acquise à l'aide du balayage de tomographie.

2. Procédé selon la revendication 1, **caractérisé en ce que** les spécifications de fabrication comprennent une sélection de matériaux (140) concernant un ou plusieurs matériaux pour un élément partiel respectif et la sélection de matériaux (140) comprend un ou plusieurs des matériaux suivants : matériau à mémoire de forme, matériau à auto-assemblage, matériau à gradient, muscles artificiels et/ou parties de tissu, de préférence peau, muqueuse, périoste, tissu mou.

3. Procédé selon l'une des revendications 1 ou 2 précédentes, **caractérisé en ce que** les spécifications de fabrication comprennent une sélection d'un ou plusieurs types d'assemblage entre les éléments partiels respectifs et la sélection des types d'assemblage comprend au moins le choix entre les types d'assemblage suivants : assemblage à queue d'aronde, assemblage par broche et/ou assemblage articulé, en particulier assemblage à rotule.

4. Procédé selon l'une des revendications précédentes, **caractérisé en ce que** l'élaboration du modèle (120) est effectuée sous forme d'élaboration d'un modèle informatique tridimensionnel, de préférence selon la méthode des éléments finis.

5. Procédé selon l'une des revendications précédentes, **caractérisé en ce que** l'acquisition des données est effectuée par un procédé d'imagerie en vue d'élaborer un modèle tridimensionnel.

6. Procédé selon l'une des revendications précédentes, **caractérisé en ce que** les différents paramètres de matériau et limites de matériau, en particulier l'épaisseur de matériau, la limite d'élasticité de matériau, la limite de rupture de matériau, en particulier sous contrainte mécanique dans différentes directions, et/ou l'élasticité de matériau de zones partielles de l'implant sont pris en compte pour l'élaboration du modèle.

7. Procédé selon l'une des revendications précédentes, **caractérisé en ce que** la sélection du type d'assemblage des différents éléments partiels est effectuée à l'aide du modèle en fonction de cas de charge individuels sur l'élément partiel.

8. Procédé selon l'une des revendications précédentes, **caractérisé en ce qu'**au moins 66 % des éléments partiels, de préférence au moins 80 % des éléments partiels, sont fabriqués différemment les uns des autres en ce qui concerne leurs dimensions.

9. Procédé selon l'une des revendications précédentes, **caractérisé en ce que** les éléments partiels à l'état assemblé d'un implant et/ou d'une ostéosynthèse définissent un modèle filaire avec un volume plus grand que chaque élément partiel individuel, de préférence un modèle filaire au moins trois fois plus grand.

10. Procédé selon la revendication 10, **caractérisé en ce que** les éléments partiels à l'état assemblé de l'implant et/ou de l'ostéosynthèse définissent un modèle filaire avec un volume au moins 8 fois plus grand que chaque élément partiel individuel.

11. Procédé selon l'une des revendications précédentes, **caractérisé en ce qu'**un premier élément partiel des éléments partiels est constitué d'un premier matériau et/ou d'une première combinaison de matériaux et un deuxième élément partiel des éléments partiels est constitué d'un deuxième matériau et/ou d'une deuxième combinaison de matériaux, la densité moyenne en volume et/ou le module d'élasticité moyen en volume du premier matériau et/ou de la première combinaison de matériaux étant plus élevés, de préférence au moins 1,2 fois plus élevés, particulièrement de préférence au moins 1,5 fois plus élevés, que la densité moyenne en volume et/ou le module d'élasticité moyen en volume du deuxième matériau et/ou de la deuxième combinaison de matériaux.

12. Procédé selon l'une des revendications précédentes, **caractérisé en ce qu'**un élément partiel est constitué d'un premier matériau et d'un deuxième matériau, la densité et/ou le module d'élasticité du premier matériau étant plus élevés, de préférence au moins 1,2 fois plus élevés, particulièrement de préférence au moins 1,5 fois plus élevés, que la densité et/ou le module d'élasticité du deuxième matériau.

13. Procédé selon l'une des revendications précédentes, **caractérisé en ce qu'**un ou plusieurs éléments partiels sont constitués d'un matériau résorbable, de préférence d'une matière plastique résorbable ou de magnésium.
